# EUROPEAN PATENT APPLICATION

(11) **EP 4 092 685 A1**
(43) Date of publication of application: **23.11.2022**
(21) Application number: 21174258.0
(22) Date of filing: 18.05.2021
(51) Int. Cl.: G16H 50/20, A61B 5/1455

(54) **SYSTEM AND METHOD FOR GENERATING A VISUALIZATION OF OXYGEN LEVELS**

(71) Applicant: Koninklijke Philips N.V., 5656 AG Eindhoven (NL)
(72) Inventor: SCHOONENBERG, Gert Antonius Franciscus, 5656 AE Eindhoven (NL)
(74) Representative: Philips Intellectual Property & Standards

(57) **Abstract**

A system and method for generating a visualization of oxygen levels in muscular tissue in an anatomical structure of a subject is disclosed. Data including a location/structure of vessels in the anatomical structure and at least one oxygen level measurement of the muscular tissue are received. A visualization illustrating oxygen levels and vessel structure is generated based on the anatomical data, the at least one oxygen level measurement and interpolation of the at least one oxygen level measurement.

## Description

### FIELD OF THE INVENTION

The invention relates to the field of generating a visualization of oxygen levels, and in particular to generating a visualization of oxygen levels in muscular tissue in an anatomical structure.

### BACKGROUND OF THE INVENTION

Myocardial ischemia occurs when blood flow to the heart is reduced, preventing the heart muscle from receiving sufficient oxygen. Myocardial ischemia can have various causes, including stenosis in the coronary arteries (coronary artery disease) and a narrowing of the small blood vessels that branch off from coronary arteries to send oxygen-rich blood to the heart muscle (microvascular coronary disease).

Current methods of diagnosing myocardial ischemia rely on indirect measures, such as coronary angiography, in which contrast-enhanced X-ray images are taken of the coronary arteries. These measures do not typically detect myocardial ischemia in the case of microvascular coronary disease.

There is therefore a need for improved information about oxygen levels in the heart muscle.

### SUMMARY OF THE INVENTION

The invention is defined by the claims.

According to examples in accordance with an aspect of the invention, there is provided a processing system for generating a first visualization of oxygen levels in muscular tissue in an anatomical structure of a subject.

The processing system is configured to: receive artery location data representative of locations of arteries in the anatomical structure; receive at least one oxygen level measurement of the muscular tissue in the anatomical structure; interpolate the at least one oxygen level measurement, using the artery location data and the at least one oxygen level measurement, to predict oxygen levels throughout the muscular tissue of the anatomical structure or of a region of interest comprised in the anatomical structure; generate the first visualization of oxygen levels in the muscular tissue in relation to the arteries in the anatomical structure based on the artery location data and the predicted oxygen levels.

The invention recognizes that ischemia is best detected by measuring the result of the reduced blood supply, that is, reduced oxygen levels

By using both oxygen level measurements and artery information, the first visualization is able to identify the exact location of ischemia. The first visualization also allows a clinician to determine which arteries may have limited blood flow, causing lower oxygen levels, and which area(s) of the anatomical structure may have diseased microvasculature, in the case where no significant lesions are found in the arteries.

The data representative of locations of arteries in the anatomical structure may be generic or subject specific. Subject-specific artery information will result in a more accurate visualization. Subject-specific artery location data may be obtained using any suitable imaging method, e.g. X-ray, CT, MRI or ultrasound imaging, with or without contrast agent corresponding to the imaging modality.

The first visualization may comprise or be formed of display data that can be processed by a display device to provide a visual representation. The visual representation may visually represent the oxygen levels in the muscular tissue (of the anatomical structure) in relation to the arteries of the anatomical structure.

The processing system may also be configured to provide the generated first visualization to a display device.

In some examples, the processing system is configured to obtain measurement location data representative of the location of each oxygen level measurement in the anatomical structure, and interpolate the at least one oxygen level measurement further using the measurement location data.

In some embodiments, the anatomical structure is a heart. This system allows a clinician to assess a location of myocardial ischemia, aiding the clinician in identifying a cause of myocardial ischemia.

In some embodiments, the processing system is configured to interpolate the at least one oxygen level measurement by processing the artery location data and the at least one oxygen level measurement using a model of perfusion of the anatomical structure.

A model of perfusion of the anatomical structure may be used, in combination with an oxygen level measurement, to predict oxygen levels further up or down the bloodstream from the measurement location.

In some embodiments, the processing system is further configured to generate the first visualization by: obtaining a contour of the anatomical structure; overlaying the predicted oxygen levels on the obtained contour of the anatomical structure; and overlaying representations of the arteries over the obtained contour of the anatomical structure based on the artery location data.

This allows the first visualization to be more readily interpreted by a clinician, allowing the clinician to see the position of the subject's arteries within the anatomical structure.

The contour may be a generic contour for the anatomical structure. A contour is a visual representation of an anatomical structure, and in particular, a visual representation of the boundaries of an anatomical structure.

In some embodiments, the processing system is further configured to: identify potential diseased artery segments based on the artery location data and the at least one oxygen level measurement; and overlay an indication of a location of the potential diseased artery segments on the generated first visualization.

In this way, a clinician may be presented with the artery segments that correspond to lower oxygen levels.

In some embodiments, the processing system is further configured to: identify potential diseased micro-vessel areas based on the artery location data and the at least one oxygen level measurement; and overlay an indication of a location of the potential diseased micro-vessel areas on the generated first visualization.

In this way, a clinician may be presented with the microvascular areas that correspond to lower oxygen levels.

In some embodiments, the generated first visualization further includes an indication of a location in which no oxygen level measurement is available.

This allows a clinician to determine whether further oxygen level measurements are required and a desired location of further measurements.

In some embodiments, the at least one oxygen level measurement is based on a difference in light penetration between different wavelengths of light.

This recognizes that oxygenated blood and deoxygenated blood have different light attenuation coefficients at different wavelengths. For example, oxygenated hemoglobin absorbs more infrared light and less red light than deoxygenated hemoglobin.

There is also proposed a system for generating and displaying a first visualization of oxygen levels in muscular tissue in an anatomical structure of a subject. The system comprises: an oxygen level measurement system configured to measure at least one oxygen level in the muscular tissue of the anatomical structure based on a difference in light penetration, through the muscular tissue, between different wavelengths of light, to thereby generate at least one oxygen level measurement; the processing system described above, configured to receive the at least one oxygen level measurement of the muscular tissue in the anatomical structure from the oxygen level measurement system; and a display device, configured to receive, from the processing system, and display the generated first visualization.

In some embodiments, the oxygen level measurement system comprises: a device configured to emit at least two, different wavelengths of light; a sensor (or detector) configured to measure light, to thereby generate light measurements; and a processor configured to calculate at least one oxygen level measurement based on light measurements from the sensor (or detector).

This system allows oxygen levels to be measured based on the difference in light penetration between different wavelengths of light.

In some embodiments, the device and sensor are configured to be positionable such that the muscular tissue is located between the device and sensor.

In this way, the sensor measures light that is transmitted though the muscular tissue. The device and/or sensor may be mounted, for instance, upon an intravenous or interventional device configured to be inserted into the anatomical structure, e.g. into arteries within the anatomical structure.

In some embodiments, the device is configured to, during emission of at least two wavelengths of light, be positioned on a same side of the muscular tissue as the sensor.

In this way, the sensor measures light reflected from the muscular tissue. This allows oxygen levels to be measured where the muscular tissue is too thick for the emitted wavelengths of light to penetrate through the muscular tissue or measuring at two sides of the muscular tissue is clinically difficult.

In some embodiments, the system further comprises a user input device, wherein the processing system is further configured to: generate a second visualization of oxygen levels in the muscular tissue in response to a user input received at the user input device, wherein the second visualization illustrates the oxygen levels in the muscular tissue from a different view to a view provided by the first visualization; and output the second visualization to the display device.

This recognizes that a single view of the anatomical structure may not provide a clinician with all the required information relating to oxygen levels in muscular tissue of the anatomical structure. Allowing the clinician to change the view gives the clinician an opportunity to build a more complete picture of oxygen levels in the anatomical structure.

According to another aspect of the invention, there is provided a computer-implemented method for generating a first visualization of oxygen levels in muscular tissue in an anatomical structure of a subject.

The computer-implemented method comprises: receiving data representative of locations of arteries in the anatomical structure; receiving at least one oxygen level measurement of the muscular tissue in the anatomical structure; and interpolating the at least one oxygen level measurement, using the artery location data and the at least one oxygen level measurement, to predict oxygen levels throughout the muscular tissue of the anatomical structure or of a region of interest comprised in the anatomical structure; and generating the first visualization of oxygen levels in the muscular tissue in relation to the arteries in the anatomical structure based on the artery location data and the predicted oxygen levels.

There is also proposed a computer program product comprising computer program code means which, when executed on a computer device have a processing system, cause the processing system to perform all of the steps of the method described above.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates a system for generating and displaying a visualization of oxygen levels in muscular tissue in an anatomical structure of a subject, according to an embodiment of the invention;
Figure 2 illustrates the hemoglobin absorption spectra for oxygenated hemoglobin and deoxygenated hemoglobin;
Figure 3 illustrates an example visualization of myocardial oxygen levels in the heart of a healthy subject;
Figure 4 illustrates an example visualization of myocardial oxygen levels in the heart of a subject having ischemia in the apex of the heart;
Figure 5 illustrates another example visualization of myocardial oxygen levels in the heart of a subject having ischemia in the apex of the heart;
Figure 6 illustrates an alternative oxygen measurement system to the oxygen level measurement system shown in Figure 1; and
Figure 7 illustrates a computer-implemented method for generating a visualization of oxygen levels in muscular tissue in an anatomical structure of a subject, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

According to a concept of the invention, there is proposed a system and method for generating a visualization of oxygen levels in muscular tissue in an anatomical structure of a subject. Data including a location/structure of arteries in the anatomical structure and at least one oxygen level measurement of the muscular tissue are received. A visualization illustrating oxygen levels and artery structure is generated based on the artery data, the at least one oxygen level measurement and interpolation of the at least one oxygen level measurement. Although the examples are described by mentioning arteries in the embodiments, the invention is applicable also in general for vessels, including veins, which are surrounded or are adjacent to muscular tissue. Besides predicting oxygen levels throughout the muscular tissue of the anatomical structure is also contemplated predicting oxygen levels throughout the muscular tissue of a region of interest comprised in the anatomical structure. In any of the embodiments the region of interest may be selected by the user of the system by a user interface (e.g. pointer, touch screen) from the anatomical structure.

Embodiments are at least partly based on the realization that a direct visualization of oxygen levels in muscular tissue of an anatomical structure in relation to artery anatomy shows the exact location of ischemia in the anatomical structure, and may be used to identify arteries and/or microvasculature with limited blood flow causing lower oxygen levels.

Illustrative embodiments may, for example, be employed in diagnostic and interventional procedures, such as percutaneous coronary diagnostic and interventional procedures, peripheral vascular procedures and neurological procedures.

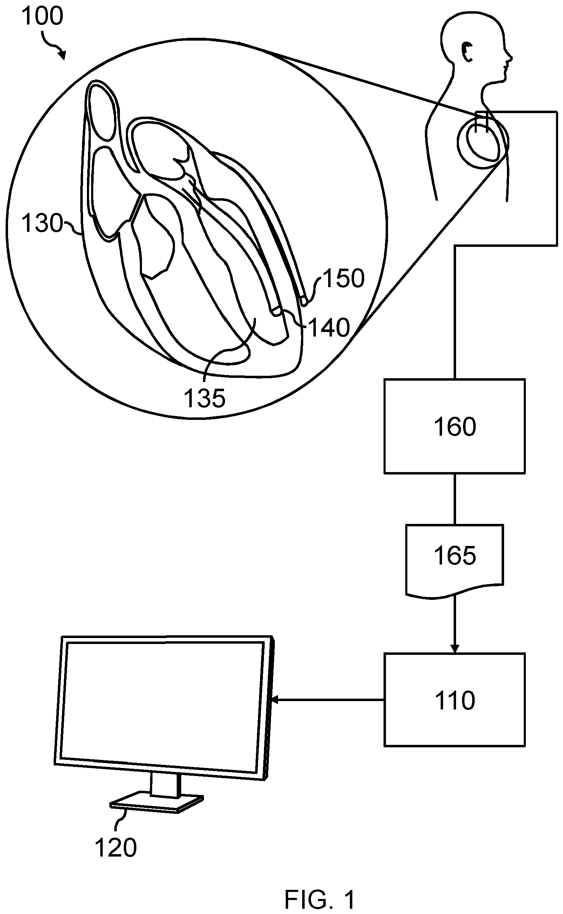

Figure 1 illustrates a system 100 for generating and displaying a visualization of oxygen levels in muscular tissue in an anatomical structure of a subject, according to an embodiment of the invention. The system comprises an oxygen level measurement system, a processing system 110 and a display device 120. The processing system 110 is, itself, an embodiment of the invention.

In Figure 1, the anatomical structure is a heart 130, and the system generates and displays a visualization of myocardial oxygen levels. However, the system may be used for generating and displaying a visualization of oxygen levels in any suitable anatomical structure of a subject. For example, the system may be used in peripheral vascular and neurological procedures.

The oxygen level measurement system is configured to measure at least one oxygen level, i.e. generate at least one sample, in muscular tissue in an anatomical structure based on a difference in light penetration between different wavelengths of light through the muscular tissue. This is based on the recognition that oxygenated blood and deoxygenated blood have different light attenuation coefficients at different wavelengths. Each measured oxygen level may be obtained at a different location within the anatomical structure.

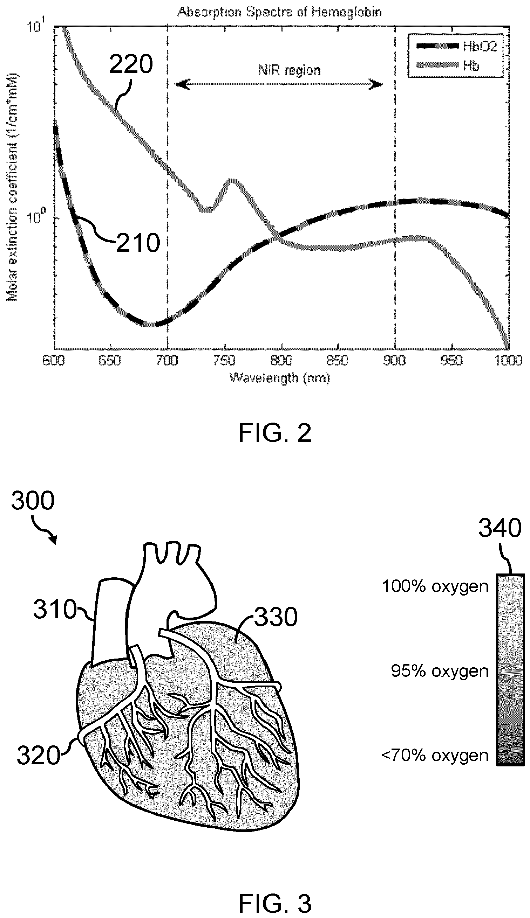

Figure 2 illustrates the hemoglobin absorption spectrum for oxygenated hemoglobin 210 and the hemoglobin absorption spectrum for deoxygenated hemoglobin 220 according to one study. As Figure 2 shows, oxygenated hemoglobin absorbs more infrared light (e.g. with a wavelength of 940 nm) and allows more red light (e.g. with a wavelength of 660 nm) to pass through compared with deoxygenated hemoglobin.

Absorption of light at a particular wavelength therefore differs for blood with different levels of oxygenation. An algorithm may be used to calculate oxygen levels based on measurements of the amount of light absorbed at different wavelengths. This principle is used in existing pulse oximeters that measure oxygen levels in the blood at, for example, a fingertip or earlobe.

The inventors have recognized that this principle may also be used to measure the oxygen levels of other tissue, for example, myocardial tissue. Research has shown that the left ventricle wall thickness has a mean thickness of 6-12 mm (see, for example, Lee et al. (2013), "Left Ventricular Wall Thickness and the Presence of Asymmetric Hypertrophy in Healthy Young Army Recruits", Circulation: Cardiovascular Imaging, Vol. 6, No. 2, pp. 262-267).

At such thicknesses, wavelengths of 660 nm and 940 nm may be used to measure oxygen levels in left ventricular wall tissue, though penetration may be less reliable for 660 nm light in thicker left ventricle walls. Higher wavelengths penetrate deeper into muscular tissue, so using wavelengths of, for example, 940 nm and 1200 nm will lead to better tissue penetration. The effective attenuation coefficients for oxygenated blood and deoxygenated blood are more similar at higher wavelengths.

Returning to Figure 1, the oxygen level measurement system comprises a device 140 configured to emit at least two, different wavelengths of light, a sensor 150 configured to measure light, and a processor 160 configured to calculate at least one oxygen level measurement based on light measurements from the sensor.

The device 140 may, for example, comprise one or more LEDs, and the sensor 150 used may be a light-sensitive sensor, the precise configuration of which may depend on the wavelengths of light emitted by the device. For example, if the device is configured to emit light of at least two, different wavelengths within a wavelength range of 660-940 nm, a silicon (Si) sensor may be suitable for measuring the light; if the device is configured to emit light of at least two, different wavelengths within a wavelength range of 660-1800 nm, a mercury cadmium telluride (HgCdTe) sensor may be suitable for measuring the light; and if the device is configured to emit light of at least two, different wavelengths within a wavelength range of 1000-1350 nm, an indium gallium arsenide (InGaAs) sensor may be suitable for measuring the light.

In the scenario illustrated by Figure 1, the device 140 is positioned in the left ventricle 135 of the subject's heart 130, while the sensor 150 is positioned in a coronary artery, so that the left ventricle wall is between the device and the sensor. The sensor is thus configured to measure light that is transmitted through the left ventricle wall. The device and sensor are each mounted on a catheter or wire (e.g. guidewire) in order to allow them to be inserted into the subject's body and moved to the desired position (for example, along the aorta to the heart).

Figure 1 illustrates only one example of a suitable oxygen level measurement system, but alternative oxygen level measurement systems that measure at least one oxygen level in muscular tissue in an anatomical structure based on a different in light penetration between different wavelengths of light through the muscular tissue are also envisaged. Further examples of suitable oxygen level measurement systems are described in more detail below.

In some embodiments, the system 100 further comprises an interface module (not shown in Figure 1) to which the catheter(s) and/or wire(s), on which the device and sensor are mounted, are connected. The processor 160 is then connected to the interface module. Alternatively, the catheter(s) and/or wire(s), on which the device and sensor are mounted, may be connected to a module that comprises the processor 160.

The processor 160 calculates at least one oxygen level measurement 165 (i.e. "sample") based on light measurements from the sensor using the difference in effective attenuation coefficients for oxygenated blood and deoxygenated blood at the wavelengths emitted by the device 140, as described above.

The processor 160 may also be configured to obtain/generate measurement location data that identifies, for each oxygen level measurement, a location/position of that oxygen level measurement within the anatomical model. The position of each oxygen level measurement (within the anatomical structure) may be identifiable, e.g. determined automatically by tracking the device and/or sensor or indicated by a user, e.g. by marking on a model of the anatomical structure or otherwise identifying the location (e.g. using a textual input).

One example of mechanisms for tracking a device within the anatomical structure is the approach suggested by US 5,983,126A, which makes uses of orthogonal alternating currents injected into a subject to track the location of a device in the subject. Other approaches are suggested by US 7,158,754 B2, US 4,173,228 and Zhou, Jun, Leonid Zamdborg, and Evelyn Sebastian. "Review of advanced catheter technologies in radiation oncology brachytherapy procedures." Cancer management and research 7 (2015): 199.

These approaches for tracking or otherwise determining a location of a device within an anatomical structure are well established in the prior art. Of course, the system 100 may comprise a device/sensor tracking system (not shown) which can be used to track or determine a position/location of the device/sensor within the anatomical structure.

The processing system 110 receives the at least one oxygen level measurement 165 from the processor 160 and generates at least a first visualization of oxygen levels in the myocardial tissue based on the at least one oxygen level measurement. In some embodiments, the processor 160 may be part of the processing system 110 (i.e. a single processing system may both calculate the at least one oxygen level measurement and generate a visualization of oxygen levels in the muscular tissue).

The processing system 110 also receives artery location data representative of locations of arteries in the anatomical structure. The artery location data may be generic data, data generated from a model, or subject-specific data. The artery location data may, for example, be 2D, 3D or 4D. Methods for obtaining subject-specific artery location data are well known, and include imaging methods such as X-ray, CT, MRI or ultrasound imaging with or without contrast agent corresponding to the respective imaging modality. In some examples, images produced from such methods undergo further processing to identify the artery location data, e.g. undergo segmentation or the like.

The processing system 110 then interpolates the at least one oxygen level measurement, using the artery location data and the at least one oxygen level measurement, to predict oxygen levels throughout the muscular tissue.

Interpolation methods are well known, and any suitable interpolation method may be used to predict the oxygen levels throughout the muscular tissue. For example, where there are sufficient oxygen level measurements, a simple bicubic interpolation may be used. In other examples, where there are not enough measurement points for the use of bicubic interpolation, more sophisticated methods may be used. In some examples, the interpolation may be based on one or more simulated models of perfusion of the anatomical structure (e.g. using classic Navier-Stokes equations). In some examples, an artificial intelligence (AI) network may be trained to predict the oxygen levels based on the measured samples.

The processing system 110 may further use measurement location data to perform the interpolation.

In particular, the processing system 110 may use a position/location of each measured oxygen level (i.e. each oxygen level sample) in order to perform the interpolation based on the artery location data. An interpolation may comprise positioning known oxygen levels at a particular position within a virtual anatomical model, and interpolating between the different oxygen levels based on the artery location data (with respect to the virtual anatomical model). Thus, the positional relationship between the measured oxygen levels and the arteries (from the artery location data) can be taken into account when performing the interpolation. This improves the accuracy of the interpolation.

In other words, the processing system may be configured to obtain measurement location data representative of the location of each oxygen level measurement in the anatomical structure, and interpolate the at least one oxygen level measurement further using the measurement location data.

Thus, in some embodiments, the step of receiving at least one oxygen level measurement may also comprise a step of receiving a location of each at least one oxygen level measurement, for use in performing interpolation. The location can be derived, for example, by an automated process that tracks the location of the device/sensor within the anatomical structure (approaches for which are well known in the art) and/or using a user input to indicate a location of each oxygen level measurement within the anatomical structure (e.g. by marking upon a visual representation of an anatomical structure).

The processing system 110 generates the first visualization of oxygen levels in the muscular tissue in relation to the arteries in the anatomical structure based on the artery location data and the predicted oxygen levels. The first visualization may comprise an overlay (e.g. a colored overlay) on a contour of the anatomical structure. For example, the processing system may obtain a contour of the anatomical structure (e.g. a generic 2D heart contour), and overlay the predicted oxygen levels and representations of the arteries, based on the artery location data, on the obtained contour. The overlay may, for example, use different colors to represent different oxygen levels, such as green for 100% oxygen, yellow/orange for lower oxygen levels and red for very low oxygen levels (e.g. <70% oxygen), or different levels of shading of a single color (e.g. grayscale).

In some embodiments, the first visualization may include additional information, such as: an indication of each measurement location (e.g. based on the measurement location data), an indication of a location of potential diseased artery segments, an indication of a location of potential diseased micro-vessel areas, and/or an indication of a location in which no oxygen level measurement is available.

In some examples, the processing system 110 overlays an indication of a measurement location corresponding to the location within the anatomical structure at which the oxygen level measurement was obtained for each of the at least one oxygen level measurement. The measurement location may be determined based on the locations of the device 140 and sensor 150, which may be determined by any suitable method of determining a position of a device inside a body (e.g. magnets, x-ray imaging, response of an electrode on the device to crossing electric fields and so on).

In some examples, the processing system 110 may identify one or more potential diseased artery segments based on the artery location data and the at least one oxygen level measurement (or the predicted oxygen levels), and overlay an indication of a location of the identified potential diseased artery segment(s) on the first visualization. Potential diseased artery segments may be identified by identifying artery segments around or beyond which the oxygen level is determined to be below a threshold (e.g. below 95% oxygen) as potential diseased artery segments.

Similarly, the processing system 110 may identify one or more potential diseased micro-vessel areas based on the artery location data and the at least one oxygen level measurement (or the predicted oxygen levels), and overlay an indication of a location of the identified potential diseased micro-vessel area(s) on the first visualization. Potential diseased micro-vessel areas may be identified by identifying areas of the anatomical structure in which the oxygen level is determined to be below a threshold (e.g. below 95% oxygen) as potential diseased micro-vessel areas.

In some examples, the processing system 110 may determine that interpolation cannot be used to reliably predict oxygen levels in the entire anatomical structure, and overlay an indication that no oxygen level measurement is available on the first visualization at one or more locations at which it is determined that an oxygen level cannot be reliably predicted. For instance, if a color overlay is used to illustrate oxygen levels in the muscular tissue, areas in which no oxygen levels are available may be grayed out.

Once the processing system 110 has generated the first visualization, the processing system provides the generated first visualization to the display device 120, which is configured to receive and display the first visualization.

In some embodiments, the system 100 may further comprise a user input device (not shown in Figure 1), connected to the processing system. The user input device may be used to receive a user instruction to generate a second visualization of oxygen levels in muscular tissue in the anatomical structure.

For example, where the first visualization is based on 3D data, the processor may be configured to generate, in response to a user input received at the user input device, a second visualization that illustrates the oxygen levels in the muscular tissue from a different view to a view provided by the first visualization. The first visualization may, for example, be generated to illustrate the oxygen levels from a predefined view (e.g. a view found to be typically a most useful view for visualizing oxygen levels in the anatomical structure), and the second visualization may be from a different predefined view, or from a view determined by the received user input.

In another example, the processing system may generate (unprompted by user input) a plurality of visualizations of oxygen levels in the muscular tissue, each from a different view of the anatomical structure, and user input from the user input device may be used to determine which of these plurality of visualizations is displayed at the display device 120 (e.g. the user input device may allow a user to rotate the displayed visualization through different views).

The system 100 may further comprise an export module (not shown in Figure 1) to use the data in (semi-)automated reports.

Figure 3 illustrates an example visualization 300 of myocardial oxygen levels in the heart of a healthy subject (i.e. a subject with no ischemia). The visualization may be generated using the system 100 illustrated in Figure 1.

The visualization 300 comprises a generic 2D heart contour 310, an artery overlay 320 and an oxygen level overlay 330. The artery overlay illustrates the structure of the arteries, while the oxygen level overlay is colored according to oxygen level. The visualization is accompanied by a key 340 or legend that illustrates which colors correspond to which oxygen levels. As Figure 3 illustrates a visualization of a healthy heart, the oxygen level overlay 330 is a solid color indicating 100% oxygen throughout the heart.

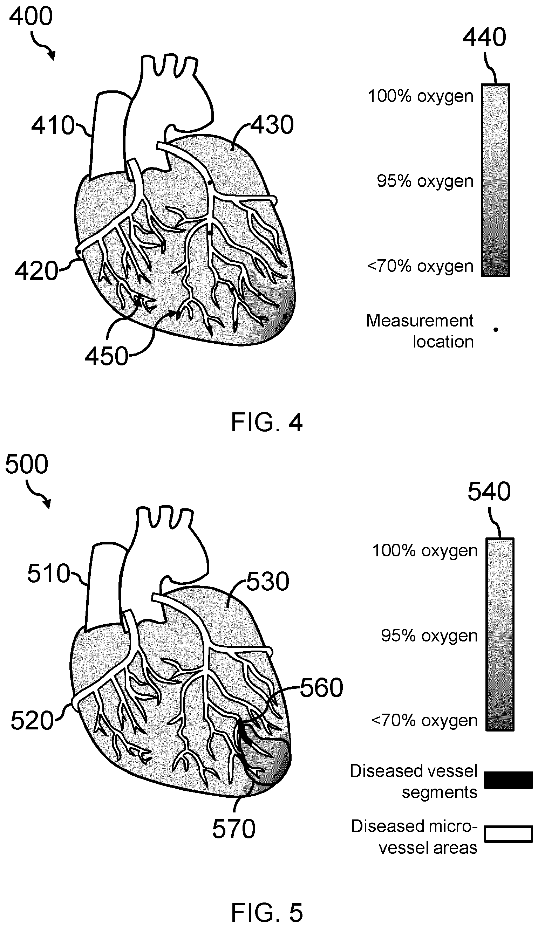

Figure 4 illustrates an example visualization 400 of myocardial oxygen levels in the heart of a subject having ischemia in the apex of the heart (the lower right portion of the visualization 400). The visualization is similar to the visualization 300 in Figure 3, with heart contour 410, artery overlay 420 and oxygen level overlay 430, but the visualization 400 additionally includes indications of measurement locations 450. The oxygen level overlay 430 varies in shading in the lower right portion, indicating that the oxygen levels are lower in this portion.

Figure 5 illustrates another example visualization 500 of myocardial oxygen levels in the heart of the same subject as Figure 4. The visualization is similar to the visualization 400 in Figure 4, with heart contour 510, artery overlay 520 and oxygen level overlay 530, but the visualization 500 additionally includes indications of potential diseased artery segments 560 and indications of potentially diseased micro-vessel segment areas 570.

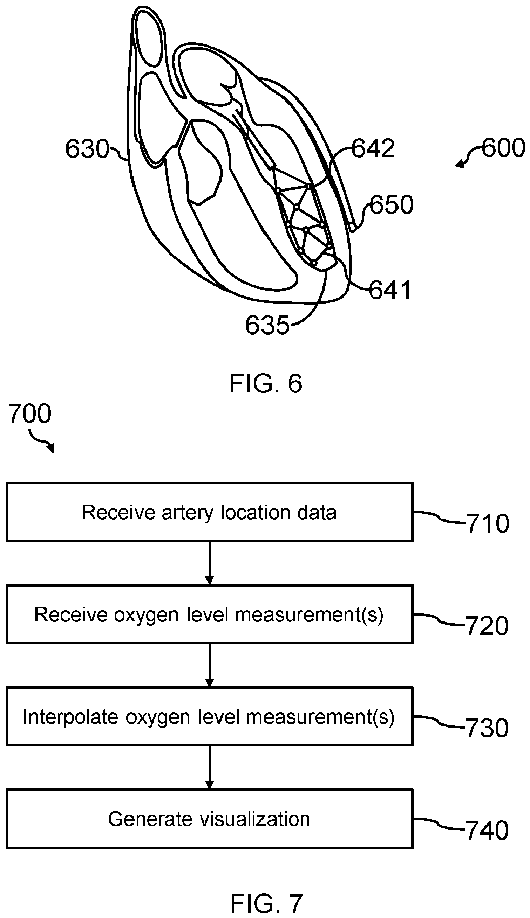

Figure 6 illustrates an alternative oxygen measurement system 600 to the oxygen level measurement system shown in Figure 1. The oxygen measurement system is similar to the oxygen level measurement system of Figure 1, with a sensor 650 mounted on a wire and positioned in a coronary artery, but instead of a single light emitting device, a self-expanding mesh 641 with multiple light sources 642 is used.

The self-expanding mesh 641 may, for example, be a nitinol mesh such as those used in self-expanding stents or valves. This system allows light to be emitted through a larger surface area of the muscular tissue, and may also aid in positioning the light source(s) close to the muscular tissue.

For example, in Figure 6, the oxygen level measurement system 600 is used to measure oxygen levels in a subject's heart 630. The self-expanding mesh 641 expands to fill the left ventricle 635, which makes it easy to position the light sources 642 at the left ventricle wall. It is desirable for the light source(s) to be positioned close to the left ventricle wall to reduce the likelihood of the oxygen level measurements) including measurements of oxygen levels in the blood in the left ventricle.

The use of multiple light sources means that it is easier to find a position at which the sensor can detect light transmitted through the left ventricle wall and allows multiple oxygen level measurements to be taken while adjusting only the position of the sensor 650.

Both Figure 1 and Figure 6 show an oxygen level measurement system in which the light is emitted from the left ventricle and detected from a coronary artery, but other examples may be the other way around (e.g. light is emitted from a coronary artery and detected from the left ventricle). Other positions in the heart such that light is emitted from one side of a section of myocardial tissue and detected having passed through the myocardial tissue may also be used.

In yet other examples, reflective measurements of light may be used to measure oxygen levels of muscular tissue instead of transmission measurements. This may be achieved by positioning a light emitting device and a sensor on the same side of the muscular tissue. For example, the device and sensor may be mounted on a single interventional device such as a catheter and positioned in either the left ventricle or a coronary artery in order to measure oxygen levels in left ventricular wall tissue.

Oxygen level measurement systems that measure oxygen levels by receiving the reflections of emissions of light of at least two different wavelengths may be used to measure oxygen levels in muscular tissue that is too thick to obtain transmission measurements (i.e. muscular tissue that has a thickness similar to or greater than the penetration depth of at least one of the emitted wavelengths).

Such systems may also allow oxygen levels to be measured in locations at which it would be clinically difficult to position a light emitting device and a sensor on opposing sides of muscular tissue.

In some examples, where a device and/or sensor is positioned in an artery, the device and/or sensor may be mounted on a catheter having a balloon to prevent blood in the artery that might affect the oxygen level measurement.

In some examples, the device and sensor (or the catheter(s)/wire(s) on which they are mounted) are equipped with magnets to aid in positioning the device and sensor inside the body. The magnets may be electromagnets to allow them to be turned on and off.

In other examples, the device and sensor may be equipped with electrodes configured to (electrically) respond to crossing electric fields induced into the anatomical structure. This allows tracking of the position of the device and/or sensor, e.g. as the response of the electrode will change depending upon its position within the crossing electric fields.

In some examples, the oxygen level measurement system may be configured to provide an indication that the device and sensor are suitably positioned for obtaining a measurement (e.g. based on position information from magnets on the device and sensor).

In some examples, the oxygen level measurement system may be configured to provide an indication of a quality of an obtained oxygen level measurement. A quality of an obtained oxygen level measurement may be determined based on a distance between and relative position of the device and sensor.

The indication that the device and sensor are suitably positioned for obtaining a measurement and/or the indication of a quality of an obtained oxygen level measurement may be output to a display device, such as the display device 120 of Figure 1.

The examples above describe measuring and visualizing oxygen levels in myocardial tissue in a subject's heart; however, the systems and methods described herein may also be used to measure and/or visualize oxygen levels in muscular tissue in other anatomical structures.

For example, oxygen levels in muscular tissue between two large blood vessels, e.g. in a subject's leg, may be measured by positioning a device configured to emit light of at least two, different wavelengths in one of the two large blood vessels and a sensor configured to detect light transmitted through the muscular tissue in the other of the two large blood vessels. A visualization of oxygen levels in the muscular tissue between the two large blood vessels may aid in diagnosing peripheral microvascular disease.

In another example, oxygen levels of muscular tissue close to a subject's skin may be measured by positioning a light emitting device against the skin and a sensor in a blood vessel on the other side of the muscular tissue (or vice versa).

Figure 7 illustrates a computer-implemented method 700 for generating a visualization of oxygen levels in muscular tissue in an anatomical structure of a subject, according to an embodiment of the invention.

The method 700 begins with step 710, in which anatomical data is received, e.g. in which locations of arteries in the anatomical structure can be identified.

At step 720, at least one oxygen level measurement of the muscular tissue in the anatomical structure is received. Step 720 may also comprise obtaining measurement location data representative of locations of each measurement in the anatomical structure.

At step 730, the at least one oxygen level measurement is interpolated, using the anatomical data (e.g. artery location data) and the at least one oxygen level measurement (and optionally, the measurement location data), to predict oxygen levels throughout the muscular tissue of the anatomical structure. Approaches for performing interpolation have been previously described.

At step 740, a first visualization of oxygen levels in the muscular tissue in relation to the anatomical structure (e.g. to the arteries in the anatomical structure) is generated based on the anatomical data (e.g. artery location data) and the predicted oxygen levels.

It will be understood that the disclosed methods are computer-implemented methods. As such, there is also proposed a concept of a computer program comprising code means for implementing any described method when said program is run on a processing system.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processors may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A processing system (110) for generating a visualization (300, 400, 500) of oxygen levels in muscular tissue in an anatomical structure (130, 630) of a subject, the processing system being configured to:
receive vessel location data representative of locations of vessels in the anatomical structure;
receive at least one oxygen level measurement (165) of the muscular tissue in the anatomical structure;
interpolate the at least one oxygen level measurement, using the vessel location data and the at least one oxygen level measurement, to predict oxygen levels throughout the muscular tissue of the anatomical structure; and
generate the visualization of oxygen levels in the muscular tissue in relation to the vessels in the anatomical structure based on the vessel location data and the predicted oxygen levels.

2. The processing system (110) of claim 1, wherein the processing system is further configured to:
obtain measurement location data representative of the location of each oxygen level measurement in the anatomical structure,
interpolate the at least one oxygen level measurement further using the measurement location data.

3. The processing system (110) of claim 1 or 2, wherein the anatomical structure (130, 630) is a heart.

4. The processing system (110) of any of claims 1 to 3, wherein the processing system is configured to interpolate the at least one oxygen level measurement (165) by processing the vessel location data and the at least one oxygen level measurement using a model of perfusion of the anatomical structure.

5. The processing system (110) of any of claims 1 to 4, wherein the processing system is further configured to generate the visualization (300, 400, 500) by:
obtaining a contour (310, 410, 510) of the anatomical structure;
overlaying the predicted oxygen levels (330, 430, 530) on the obtained contour of the anatomical structure; and
overlaying representations of the vessels (320, 420, 520) over the obtained contour of the anatomical structure based on the vessel location data.

6. The processing system (110) of any of claims 1 to 5, wherein the processing system is further configured to:
identify potential diseased vessel segments based on the vessel location data and the at least one oxygen level measurement (165); and
overlay an indication of a location of the potential diseased vessel segments (560) on the generated visualization (300, 400, 500).

7. The processing system (110) of any of claims 1 to 6, wherein the processing system is further configured to:
identify potential diseased micro-vessel areas based on the vessel location data and the at least one oxygen level measurement (165); and
overlay an indication of a location of the potential diseased micro-vessel areas (570) on the generated visualization (300, 400, 500).

8. The processing system (110) of any of claims 1 to 7, wherein the generated visualization (300, 400, 500) further includes an indication of a location in which no oxygen level measurement is available.

9. The processing system (110) of any of claims 1 to 8, wherein the at least one oxygen level measurement (165) is based on a difference in light penetration between different wavelengths of light.

10. A system (100) for generating and displaying a visualization (300, 400, 500) of oxygen levels in muscular tissue in an anatomical structure (130, 630) of a subject, the system comprising:
an oxygen level measurement system (600) configured to measure at least one oxygen level in the muscular tissue of the anatomical structure based on a difference in light penetration, through the muscular tissue, between different wavelengths of light, to thereby generate at least one oxygen level measurement (165);
the processing system (110) of any of claims 1 to 9, configured to receive the at least one oxygen level measurement of the muscular tissue in the anatomical structure from the oxygen level measurement system; and
a display device (120), configured to receive, from the processing system, and display the generated visualization.

11. The system (100) of claim 10, wherein the oxygen level measurement system (600) comprises:
a device (140) configured to emit at least two, different wavelengths of light;
a sensor (150, 650) configured to measure light, to thereby generate light measurements; and
a processor (160) configured to calculate at least one oxygen level measurement (165) based on light measurements from the sensor.

12. The system (100) of claim 11, wherein:
the device (140) and sensor (150, 650) are configured to be positionable such that the muscular tissue is located between the device and sensor; or
the device (140) is configured to, during emission of at least two wavelengths of light, be positioned on a same side of the muscular tissue as the sensor (150, 650).

13. The system (100) of any of claims 9 to 12, further comprising a user input device, wherein the processing system (110) is further configured to:
generate a further visualization of oxygen levels in the muscular tissue in response to a user input received at the user input device, wherein the further visualization illustrates the oxygen levels in the muscular tissue from a different view to a view provided by the visualization (300, 400, 500); and
output the further visualization to the display device (120).

14. A computer-implemented method (700) for generating a visualization (300, 400, 500) of oxygen levels in muscular tissue in an anatomical structure (130, 630) of a subject, the computer-implemented method comprising:
receiving data representative of locations of vessels in the anatomical structure;
receiving at least one oxygen level measurement (165) of the muscular tissue in the anatomical structure;
interpolating the at least one oxygen level measurement, using the vessel location data and the at least one oxygen level measurement, to predict oxygen levels throughout the muscular tissue of the anatomical structure; and
generating the visualization of oxygen levels in the muscular tissue in relation to the vessels in the anatomical structure based on the vessel location data and the predicted oxygen levels.

15. A computer program product comprising computer program code means which, when executed on a computer device have a processing system, cause the processing system to perform all of the steps of the method (700) according to claim 14.
